(19) Europäisches Patentamt
European Patent Office
Office européen des brevets

(11) Veröffentlichungsnummer: **0 040 358**
**B1**

# (12) EUROPÄISCHE PATENTSCHRIFT

(45) Veröffentlichungstag der Patentschrift: **19.01.83**

(51) Int. Cl.³: **C 07 C 147/02,** C 07 C 147/06, C 07 C 147/08 // C09B3/78

(21) Anmeldenummer: **81103435.4**

(22) Anmeldetag: **06.05.81**

## (54) Verfahren zur Herstellung von Sulfonylcarbonsäuren.

(30) Priorität: **16.05.80 DE 3018822**
**21.06.80 DE 3023331**

(43) Veröffentlichungstag der Anmeldung:
**25.11.81 Patentblatt 81/47**

(45) Bekanntmachung des Hinweises auf die Patenterteilung:
**19.01.83 Patentblatt 83/3**

(84) Benannte Vertragsstaaten:
**CH DE FR GB IT LI**

(56) Entgegenhaltungen:
**US-A-3 441 614**
**Chemical Abstracts Band 62, Nr. 5, 1. März 1965 Columbus, Ohio, USA M. ASSCHER et al. »Chlorine-activation by redox-transfer. IV. The addition of sulfonyl chlorides to vinyl monomers and other olefins« Spalte 5216 a—c & J. Chem. Soc. 1964, Nr. 12, Seiten 4962 bis 4971**
**Chemical Abstracts Band 65, Nr. 8, 10. Oktober 1966, Columbus, Ohio, USA I. L. KNUNYANTS et al. »Structure of addition products of phenylsulfenyl chloride and acrylic acid derivatives« Spalten 12134g bis 12135b & Izv. Akad. Nauk SSSR, Ser. Khim. 1966 Nr. 6, Seiten 1075 bis 1080**

(73) Patentinhaber: **BAYER AG, Zentralbereich Patente, Marken und Lizenzen, D-5090 Leverkusen 1, Bayerwerk (DE)**

(72) Erfinder: **Schallner, Otto, Dr., Jakob-Boehme-Strasse 11, D-5000 Köln 80 (DE)**
Erfinder: **Schündehütte, Karl Heinz, Dr., Klief 75, D-5090 Leverkusen 3 (DE)**

EP 0 040 358 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Sulfonylcarbonsäuren

Die Erfindung betrifft ein neues Verfahren zur Herstellung von 3-Halogen-3-sulfonylpropionsäuren und von deren Dehydrohalogenierungsprodukten, den 3-Sulfonylacrylsäuren aus 2-Sulfonyl-bernsteinsäuren durch Umsetzung mit Halogenierungsmitteln und gegebenenfalls nachfolgende thermische Dehydrohalogenierung.

Die Darstellung einiger 3-Chlor-3-sulfonylpropionsäuren bzw. ihrer Derivate ist bekannt. So beschreiben I. L. Knunyants, M. G. Lin'Kova und N. L. Veller, Izv. Akad. Nauk. SSSR, Ser. Khim 1966 (6) 1075 die Herstellung von 3-Chlor-3-phenylsulfonylpropionsäure durch Addition von Chlorwasserstoff an 3-Phenylsulfonylacrylsäure in etherischer Lösung. Die 3-Phenylsulfonylacrylsäure erhielten sie durch Eliminierung von Chlorwasserstoff aus 2-Chlor-3-phenylsulfonylpropionsäure in wäßriger Lösung mit Hilfe von Natronlauge.

Einen ähnlichen Weg schlugen M. Asscher und D. Vofsi, J. Chem. Soc. 1964 4962 bei der Herstellung von 3-Chlor-3-methylsulfonylpropionsäuremethylester ein:

$$CH_3-SO_2-CH_2-CHCl-CN \longrightarrow CH_3-SO_2-CH{=}CH-COOH$$

$$\xrightarrow{HCl/MeOH} CH_3-SO_2-CHCl-CH_2-COOCH_3$$

Durch Verseifung von 2-Chlor-3-methylsulfonylpropionitril und anschließender Veresterung der entstandenen 3-Methylsulfonylacrylsäuren mit Methanol-Chlorwasserstoff erhielten sie den obengenannten Ester.

Es wurde nun ein neues, einfaches Verfahren zur Herstellung von 3-Halogen-3-sulfonylpropionsäuren und von 3-Sulfonylacrylsäuren gefunden, bei dem die gewünschten Carbonsäuren direkt entstehen, ohne daß die Notwendigkeit besteht, Zwischenstufen in Form von Derivaten oder Isomeren zu durchlaufen oder gar zu isolieren.

Mit der vorliegenden Erfindung wird ein neues universell anwendbares Verfahren geschaffen, das es gestattet, eine große Anzahl von verschiedenartigsten 3-Halogen-3-sulfonylpropionsäuren und von deren Dehydrohalogenierungsprodukten, den 3-Sulfonylacrylsäuren, zu synthetisieren.

Das erfindungsgemäße Verfahren zur Herstellung von Sulfonylcarbonsäuren der allgemeinen Formel

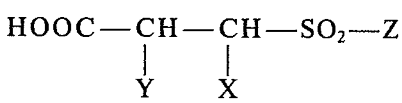

(I)

worin

X Halogen und
Y Wasserstoff oder worin X und Y zusammen für eine Kohlenstoff-Kohlenstoff-Einfachbindung stehen und worin
Z gegebenenfalls substituiertes Alkyl, gegebenenfalls substituiertes Aryl oder gegebenenfalls substituiertes Hetaryl,

ist dadurch gekennzeichnet, daß man zu einer Dicarbonsäure der Formel

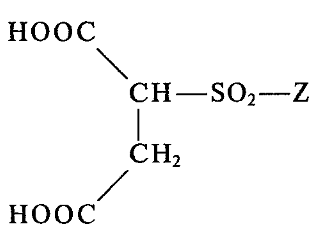

(II)

in welcher

Z die oben angegebene Bedeutung besitzt,

in einem gegenüber Halogenierungsmittel inerten Lösungsmittel bei 0 bis 80°C mindestens die äquimolare Menge eines Halogenierungsmittels gibt, und

a) zum Erhalt von Sulfonylcarbonsäuren der Formel (I), in welcher X für Halogen und Y für Wasserstoff steht, das Reaktionsprodukt aufarbeitet oder

b) zum Erhalt von Verbindungen der Formel (I), in welcher X und Y zusammen für eine Kohlenstoff-Kohlenstoff-Einfachbindung stehen, das Reaktionsprodukt auf Temperaturen von 85 bis 150° C erhitzt.

In den Formeln (I) bzw. (II) haben die Substituenten X, Y und Z vorzugsweise folgende Bedeutung:

X Chlor oder Brom und
Y Wasserstoff oder
X und Y stehen für eine Kohlenstoff-Kohlenstoff-Einfachbindung
Z gegebenenfalls durch Halogen (insbesondere Chlor), OH, CN, COOH, Alkoxy ($C_1-C_4$), Alkyl ($C_1-C_4$)sulfonyl, Phenyl, Naphthyl substituiertes Alkyl ($C_1-C_{15}$); gegebenenfalls durch Alkyl ($C_1-C_4$), Halogen, COOH, $SO_3H$, Alkoxy ($C_1-C_4$), Alkyl($C_1-C_4$)sulfonyl, $NO_2$, CN, $CF_3$, substituiertes Phenyl oder Naphthyl; gegebenenfalls durch Halogen, OH, CN, Alkyl ($C_1-C_4$), Alkoxy ($C_1-C_4$), COOH oder $SO_3H$ substituierter 5- oder 6gliedriger aromatischer Heterocyclus, wie z. B. Thienyl, Pyridyl.

Bei der erfindungsgemäßen Umsetzung bevorzugte Halogenierungsmittel sind Halogene, insbesondere Chlor und Brom.

Die Umsetzung mit dem Halogenierungsmittel erfolgt in gegenüber diesem inerten Lösungsmittel, vorzugsweise in Wasser, niederen Alkanolen oder in deren Gemischen, insbesondere mit Wasser.

Bei der Halogenierungsreaktion kann es auch vorteilhaft sein, die Umsetzung mit mehr als der äquimolaren Menge Halogenierungsmittel (bis zur 2fachen, insbesondere bis zur 1,5fachen Menge) durchzuführen. Die Reaktionstemperatur beim Herstellungsverfahren von Verbindungen der Formel (I) in welcher X für Halogen und Y für Wasserstoff steht, beträgt 0 bis 80° C, insbesondere 20 bis 65° C.

Zur Durchführung des erfindungsgemäßen Verfahrens müssen die Ausgangsverbindungen der Formel (II) nicht gesondert hergestellt und isoliert werden. Es kann sogar vorteilhaft sein, daß Reaktionsgemisch einer vorgeschalteten Reaktion, so wie es anfällt, für die weitere Umsetzung zu verwenden.

Vorgeschaltete Umsetzungen zur Herstellung von Verbindungen der Formel (II) sind beispielsweise Umsetzung von Malein- oder Fumarsäure mit einer Sulfinsäure der Formel $Z-SO_2H$, wobei Z die oben angegebene Bedeutung besitzen kann oder auch Umsetzung einer substituierten Bernsteinsäure der Formel

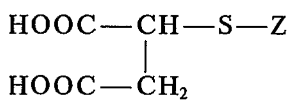

worin

Z die oben angegebene Bedeutung besitzt, mit einem geeigneten Oxydationsmittel wie z. B. $H_2O_2$, Halogene wie Chlor oder Brom.

Aus der großen Zahl, der mit Hilfe dieses Verfahrens darstellbaren 3-Halogen-3-sulfonylpropionsäuren seien die folgenden beispielhaft erwähnt:

3-Brom-3-phenylsulfonyl-, 3-Brom-3-(o-toluylsulfonyl)-,
3-Brom-3-(m-toluylsulfonyl)-, 3-Brom-3-(p-toluylsulfonyl)-,
3-Brom-3-xylylsulfonyl-, 3-Brom-3-mesityl-sulfonyl-,
3-Brom-3-(2'-chlorphenylsulfonyl)-, 3-Brom-3-(3'-chlorphenylsulfonyl)-,
3-Brom-3-(4'-chlorphenylsulfonyl)-, 3-Brom-3-(2,5'-dichlorphenylsulfonyl)-,
3-Brom-3-(3,4'-dichlorphenylsulfonyl)-, 3-Brom-3-(2'-nitrophenylsulfonyl)-,
3-Brom-3-(3'-nitrophenylsulfonyl)-, 3-Brom-3-(4'-nitrophenylsulfonyl)-,
3-Brom-3-(4'-chlor-3'-nitrophenylsulfonyl)-,
3-Brom-3-(2'-chlor-5'-nitrophenylsulfonyl)-,
3-Brom-3-(4'-chlor-2'-nitrophenylsulfonyl)-,
3-Brom-3-(5'-chlor, 2'-methylphenylsulfonyl)-,
3-Brom-3-(3-chlor, 4'-methylphenylsulfonyl)-,
3-Brom-3-(2'-methyl, 5'-nitrophenylsulfonyl)-,
3-Brom-3-(4'-methyl, 3'-nitrophenylsulfonyl)-,
3-Brom-3-(4'-cyanophenylsulfonyl)-, 3-Brom-3-(2'-methoxyphenylsulfonyl)-,
3-Brom-3-(4'-methoxyphenylsulfonyl)-,
3-Brom-3-(4'-methoxy, 2'-methylphenylsulfonyl)-,
3-Brom-3-(4-methoxy, 3'-methylphenylsulfonyl)-,
3-Brom-3-(4'-methoxy, 3'-nitrophenylsulfonyl)-,
3-Brom-3-(4'-fluorphenylsulfonyl)-,

3-Brom-3-(4′-phenylphenylsulfonyl)-, 3-Brom-(3′-carboxyphenylsulfonyl-,
3-Brom-(4′-carboxyphenylsulfonyl)-, 3-Brom-3-methylsulfonyl-,
3-Brom-3-ethylsulfonyl-, 3-Brom-3-n-propylsulfonyl-,
3-Brom-3-iso-propylsulfonyl-, 3-Brom-3-n-butylsulfonyl-,
3-Brom-3-iso-butylsulfonyl-, 3-Brom-3-tert.-butylsulfonyl-,
3-Brom-3-pentylsulfonyl-, 3-Brom-3-hexylsulfonyl-,
3-Brom-3-heptylsulfonyl-, 3-Brom-3-decylsulfonyl-,
3-Brom-3-dodecylsulfonyl-, 3-Brom-3-tert.-dodecylsulfonyl-,
3-Brom-3-chlormethylsulfonyl-,
3-Brom-3-(2′-chlorethylsulfonyl)-, 3-Brom-3-benzylsulfonyl-,
3-Brom-3-(2′-methoxyethylsulfonyl)-propionsäure,
Bis-(1-brom-2-carboxy)ethylsulfon,
2,2′-Bis-[(1″-brom-2″-carboxy)ethylsulfonyl]-diethylether,
2,2′-Bis[(1″-Brom-2″-carboxy)ethylsulfonyl]-diethylsulfon,
1,3-Bis[(1′-brom-2′-carboxy)ethylsulfonyl]-2-hydroxypropan,
3-Chlor-3-phenylsulfonyl-, 3-Chlor-3-(o-toluylsulfonyl)-,
3-Chlor-3-(m-toluylsulfonyl)-, 3-Chlor-3-(p-toluylsulfonyl)-,
3-Chlor-3-xyloylsulfonyl-, 3-Chlor-3-mesitylsulfonyl-,
3-Chlor-3-(2′-chlorphenylsulfonyl)-, 3-Chlor-3-(3′-chlorphenylsulfonyl)-,
3-Chlor-3-(4′-chlorphenylsulfonyl)-,
3-Chlor-3-(2′,5′-dichlorphenylsulfonyl)-,
3-Chlor-3-(3,4′-dichlorphenylsulfonyl)-,
3-Chlor-3-(2′-nitrophenylsulfonyl)-,
3-Chlor-3-(3′-nitrophenylsulfonyl)-, 3-Chlor-3-(4-nitrophenylsulfonyl)-,
3-Chlor-3-(4′-chlor-3′-nitrophenylsulfonyl)-,
3-Chlor-3-(2′-chlor,5′-nitrophenylsulfonyl)-,
3-Chlor-3-(4′-chlor,2′-nitrophenylsulfonyl)-,
3-Chlor-3-(5′-chlor,2′-methylphenylsulfonyl)-,
3-Chlor-3-(3-chlor,4′-methylphenylsulfonyl)-,
3-Chlor-3-(2′-methyl,5′-nitrophenylsulfonyl)-,
3-Chlor-3-(4′-methyl,3′-nitrophenylsulfonyl)-,
3-Chlor-3-(4′-cyanophenylsulfonyl)-,
3-Chlor-3-(2′-methoxyphenylsulfonyl)-,
3-Chlor-3-(4′-methoxyphenylsulfonyl)-,
3-Chlor-3-(4′-methoxy, 2′-methylphenylsulfonyl)-,
3-Chlor-3-(4′-methoxy, 3′-methylphenylsulfonyl)-,
3-Chlor-3-(2′-methoxy-4′-methylphenylsulfonyl)-,
3-Chlor-3-(4′-methoxy-3′-nitrophenylsulfonyl)-,
3-Chlor-3-(4′-fluorphenylsulfonyl)-, 3-Chlor-3-(4′-phenylphenylsulfonyl)-,
3-Chlor-(3′-carboxyphenylsulfonyl)-, 3-Chlor-(4′-carboxyphenylsulfonyl)-,
3-Chlor-3-methylsulfonyl-, 3-Chlor-3-ethylsulfonyl-,
3-Chlor-3-n-propylsulfonyl-, 3-Chlor-3-iso-propylsulfonyl-,
3-Chlor-3-n-butylsulfonyl-, 3-Chlor-3-iso-butylsulfonyl-,
3-Chlor-3-tert.-butylsulfonyl-, 3-Chlor-3-pentylsulfonyl-,
3-Chlor-3-hexylsulfonyl-, 3-Chlor-3-heptylsulfonyl-,
3-Chlor-3-decylsulfonyl-, 3-Chlor-3-dodecylsulfonyl-,
3-Chlor-3-tert.-dodecylsulfonyl-, 3-Chlor-3-chlormethylsulfonyl-,
3-Chlor-3-(2′-chlorethylsulfonyl)-, 3-Chlor-3-benzylsulfonyl-,
3-Chlor-3-(2′-methoxyethylsulfonyl)-propionsäure,
Bis-(1-Chlor-2-carboxy)ethylsulfon,
2,2′-Bis[(1″-chlor-2″-carboxy)ethylsulfonyl]-diethylether,
2,2′-Bis-[(1″-chlor-2″-carboxy)ethylsulfonyl]-diethylsulfon,
1,3-Bis-[(1′-chlor-2′-carboxy)ethylsulfonyl]-2-hydroxypropan.

Aus den so erhaltenen 3-Halogen-3-sulfonylpropionsäuren kann man durch einfaches Erwärmen mit oder ohne Lösungsmittel die entsprechenden 3-Sulfonylacrylsäuren erhalten. Die Dehydrohalogenierungsreaktion kann bei 85° — 150° C vorzugsweise bei 90° — 120° C durchgeführt werden.

Die so hergestellten Verbindungen sind wichtige Zwischenprodukte, beispielsweise für die Herstellung neuer Carbonsäurederivate, die unter anderem als Reaktivfarbstoffe dienen.

Als Beispiel für den Einsatz eines Zwischenproduktes der Formel (I) für die Herstellung eines Reaktivfarbstoffes sei aufgeführt:

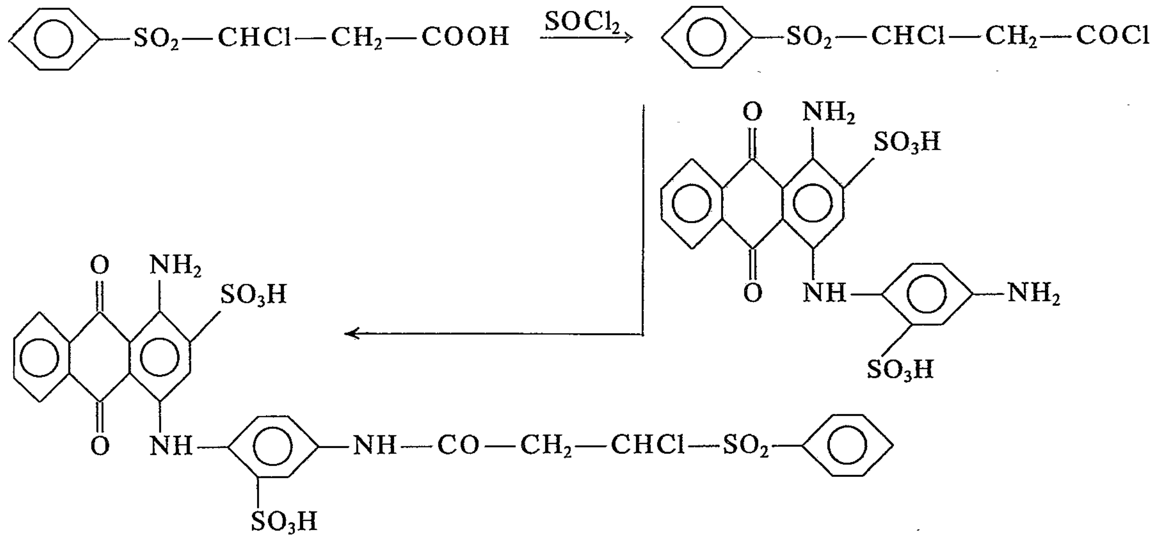


## Herstellungsbeispiele

### Beispiel 1

164 g (1 Mol) des Natriumsalzes der Benzolsulfinsäure werden in 1100 ml Wasser gelöst und mit einer Suspension von 127,6 g (11 Mol) Maleinsäure in 150 ml Wasser versetzt. Man erwärmt eine Stunde auf 90°C, kühlt auf 50°C ab und leitet bei dieser Temperatur innerhalb von 3 Stunden 85 g Chlorgas ein. Mit fortschreitender Reaktion scheidet sich eine ölige Phase ab, die im Laufe der Zeit erstarrt. Man saugt den festen Rückstand ab, wäscht mit etwas verdünnter Salzsäure und trocknet im Vakuum bei 60—80°. Ausbeute: 225 g (≙ 90% der Theorie) 3-Chlor-3-phenylsulfonylpropionsäure. $^1$H-NMR-Spektrum (Aceton-$d^6$): $\delta = 2{,}83$ (doppeltes Dublett, 1H, $I_1 = 16$ Hz, $I_2 = 10$ Hz); 3,55 (doppeltes Dublett, 1H, $I_1 = 16$ Hz, $I_2 = 3{,}5$ Hz); 5,42 (doppeltes Dublett, 1H, $I_1 = 10$ Hz, $I_2 = 3{,}5$ Hz); 7,78 (Multiplett, 3H) 8,04 (Multiplett, 2H); 10,5 (Singulett, 1H).

Verfährt man wie oben beschrieben und verwendet anstelle des Natriumbenzolsulfinats die in Spalte 2 der folgenden Tabelle angegebenen Sulfinate, so erhält man die in Spalte 3 der Tabelle angegebenen Propionsäuren mit den in Spalte 4 angegebenen NMR-Spektren in den in Spalte 5 angegebenen Ausbeuten.

| Beispiel | Sulfinate | Verfahrensprodukte (I) | $^1$H-NMR-Spektren der Verfahrensprodukte ($\delta$ in ppm von TMS) | Ausbeute in % der Theorie |
|---|---|---|---|---|
| 2 | 4-Methylbenzolsulfinat | 3-Chlor-3-(4'-methylphenylsulfonyl)-propionsäure | $\delta$ = 2,45 (Singulett, 3 H); 2,81[a]) (doppeltes Dublett, 1 H); 3,57 (doppeltes Dublett, 1 H); 5,40 (doppeltes Dublett, 1 H); 7,58 (Dublett, 2 H); 8,00 (Dublett, 2 H); 1,03 (Singulett, 1 H) | 85 |
| 3 | 4-Chlorbenzolsulfinat | 3-Chlor-3-(4'-chlorphenylsulfonyl)-propionsäure | $\delta$ = 2,80 (doppeltes Dublett, 1 H);[a]) 3,52 (doppeltes Dublett, 1 H); 5,40 (doppeltes Dublett, 2 H); 7,98 (Multiplett, 4 H); 10,35 (Singlulett, 1 H) | 70 |
| 4 | 4-Fluorbenzolsulfinat | 3-Chlor-3-(4'-fluorphenylsulfonyl)-propionsäure | $\delta$ = 2,81 (doppeltes Dublett, 1 H); 3,50[a]) (doppeltes Dublett, 1 H); 5,42 (doppeltes Dublett, 1 H); 7,49 (Triplett, 2 H); 8,12 (Multiplett, 2 H); 7,75 (breites Singulett, 1 H) | 87 |
| 5 | 3-Carboxybenzolsulfinat | 3-Chlor-3-(3'-carboxyphenylsulfonyl)-propionsäure | $\delta$ = 2,88 (doppeltes Dublett, 1 H); 3,55[a]) (doppeltes Dublett, 1 H); 7,88 (Multiplett, 1 H); 8,47 (Multiplett, 3 H); 9,64 (Singulett, 2 H) | 59 |
| 6 | 3-Chlorbenzolsulfinat | 3-Chlor-3-(3'-chlorphenylsulfonyl)-propionsäure | $\delta$ = 2,84 (doppeltes Dublett, 1 H);[a]) 3,54 (doppeltes Dublett, 1 H); 5,50 (doppeltes Dublett, 1 H); 7,86 (Multiplett, 1 H); 8,02 (Multiplett, 3 H); 9,70 (Singulett, 1 H) | 75 |
| 7 | Methylsulfinat | 3-Chlor-3-methylsulfonyl-propionsäure | $\delta$ = 2,72 (doppeltes Dublett, 1 H);[b]) 3,15 (Singulett, 3 H); 3,33 (doppeltes Dublett, 1 H); 5,58 (doppeltes Dublett, 1 H) | 35 |
| 8 | 4-Nitrobenzolsulfinat | 3-Chlor-3-(4'-nitrophenylsulfonyl)-propionsäure | $\delta$ = 2,84 (doppeltes Dublett, 1 H); 3,55[a]) (doppeltes Dublett, 1 H); 5,56 (doppeltes Dublett, 1 H); 8,45 (doppeltes Dublett, 4 H) | 50 |
| 9 | 4-Chlor-3-nitrobenzolsulfinat | 3-Chlor-3-(4'-Chlor-3'-nitrophenylsulfonyl)-propionsäure | $\delta$ = 2,88 (doppeltes Dublett, 1 H); 3,55[a]) (doppeltes Dublett, 1 H); 5,52 (doppeltes Dublett, 1 H); 8,08 (Dublett, 1 H); 8,35 (doppeltes Dublett, 1 H); 8,64 (Dublett, 1 H); 8,83 (Singulett, 1 H) | 28 |

[a]) Aceton-$d^6$,
[b]) Dimethylsulfoxid-$d^6$

## Beispiel 10

Verfährt man wie in Beispiel 1 und tropft — anstelle von Chlorgas — 175 g Brom in die Reaktionsmischung und arbeitet wie beschrieben auf, so erhält man aus einem Mol Natriumbenzolsulfinat und 1,1 Mol Maleinsäure 250 g (≙ 85% Ausbeute) 3-Brom-3-phenylsulfonylpropionsäure $^1$H-NMR-Spektrum (Aceton-$d^6$): $\delta$=2,96 (doppeltes Dublett, 1H, $I_1$=16 Hz, $I_2$=10 Hz); 3,65 (doppeltes Dublett, 1H, $I_1$=16 Hz, $I_2$=3,5 Hz); 5,48 (doppeltes Dublett, 1H, $I_1$=10 Hz, $I_2$=3,5 Hz); 7,72 (Multiplett, 3H); 8,05 (Multiplett, 2H); 9,75 (Singulett, 1H).

## Beispiel 11

226 g (1 Mol) Phenylthiobernsteinsäure werden mit 1000 ml Wasser versetzt, mit Salzsäure auf pH 1 gestellt und — zuerst bei 0—5° C, dann bei 50° C — mit insgesamt 240 g Chlor zur Reaktion gebracht. Die anfänglich nicht vollständig gelöste Carbonsäure geht während der Reaktion in Lösung. Gegen Ende der Umsetzung scheidet sich eine ölige Phase ab, die mit der Zeit fest wird. Nach der üblichen Aufarbeitung erhält man 95 g (≙ 38% Ausbeute) einer Carbonsäure, die mit der in Beispiel 1 erhaltenen 3-Chlor-3-phenylsulfonylpropionsäure identisch ist.

## Beispiel 12

Verwendet man anstelle von Phenylthiobernsteinsäure 206 g (1 Mol) n-Butylthiobernsteinsäure und verfährt sonst wie in Beispiel 11 beschrieben, so erhält man 82 g (≙ 36% Ausbeute) 3-Chlor-3-n-butylsulfonylpropionsäure $^1$H-NMR-Spektrum (Aceton-$d^6$): $\delta$=0,98 (Triplett, 3H); 1,71 (Multiplett, 4H); 2,85 (doppeltes Dublett, 1H); 3,21—3,67 (Multiplett, 3H); 5,31 (doppeltes Dublett, 1H); 9,35 (Singulett, 1H).

## Beispiel 13

190 g (0,714 Mol) Thiodibernsteinsäure werden in 700 ml Wasser suspendiert. Man versetzt mit 4,7 g (0,014 Mol) Natriumwolframat-2-hydrat und tropft innerhalb 1 h 135 ml (1,57) 35%ige Wasserstoffperoxidlösung zu. Die Temperatur wird durch Außenkühlung auf 20—25° C gehalten. Es wird 2 Stunden bei dieser Temperatur nachgerührt und anschließend bei 40° C innerhalb von 3 Stunden mit 135 g Chlor (1,9 Mol) umgesetzt. Es wird anschließend eine halbe Stunde auf 60—70° erwärmt, von ausgefallener Polywolframsäure abfiltriert und das Filtrat auf ein Drittel seines ursprünglichen Volumens eingeengt (Wasserstrahlvakuum, 60° C). Das aus dieser Lösung auskristallisierende Produkt wird abfiltriert, gewaschen und getrocknet. Ausbeute: 170 g (≙ 83%) Bis-(1-chlor-2-carboxy)-ethylsulfon. $^1$H-NMR-Spektrum (Aceton-$d^6$): $\delta$=2,93 (doppeltes Dublett, 2H); 3,51 (doppeltes Dublett, 2H); 5,75 (doppeltes Dublett, 2H); 8,80 (Singulett 2H).

## Beispiel 14

Verwendet man anstelle von Thiodibernsteinsäure 103 g (≙ 0,5 Mol) n-Butylthiobernsteinsäure und verfährt sonst wie in Beispiel 13 beschrieben, 400 ml Wasser, 3,3 g (≙ 0,01 Mol) Natriumwolframat-2-hydrat, 94 ml (≙ 1,1 Mol) 35%ig Wasserstoffperoxid-Lösung und 70 g (0,99 Mol) Chlor, so erhält man 55 g (≙ 48%) einer Carbonsäure die mit der in Beispiel 12 beschriebenen 3-Chlor-3-n-butylsulfonylpropionsäure identisch ist.

## Beispiel 15

Verwendet man anstelle von n-Butylthiobernsteinsäure 159 g (0,5 Mol) n-Dodecylthiobernsteinsäure und verfährt sonst wie in Beispiel 14 beschrieben, so erhält man 152 g 3-Chlor-3-n-dodecylsulfonylpropionsäure (≙ 88%). $^1$H-NMR-Spektrum (Aceton-$d^6$): $\delta$=0,88 (Multiplett, 3H) 1,12—2,15 (Multiplett, breit, 20H); 2,90 (doppeltes Dublett, 1H); 3,20—3,70 (Multiplett, 3H); 5,30 (doppeltes Dublett, 1H); 9,85 (Singulett, 1H).

## Beispiel 16

50 g (0,2 Mol) 3-Chlor-3-phenylsulfonylpropionsäure werden in 50 ml Wasser suspendiert und unter Rühren 5 Stunden unter Rückfluß erhitzt. Die entstandene ölige Phase erstarrt beim Erkalten. Der

Kristallbrei wird abgesaugt und getrocknet. Ausbeute: 42 g (≙ 98%) 3-Phenylsulfonylacrylsäure. $^1$H-NMR (Dimethylsulfoxid-$d^6$): $\delta = 6{,}76$ (Dublett, 1H, J = 16 Hz); 7,63 (Dublett, 1H, J = 16 Hz); 7,72 (Multiplett, 3H); 7,93 (Multiplett, 2H); 8,30 (Singulett, 1H).

**Patentansprüche**

1. Verfahren zur Herstellung von Sulfonylcarbonsäuren der allgemeinen Formel

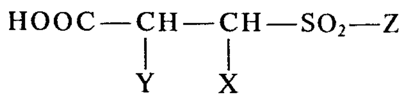

(I)

worin

X = Halogen und
Y = Wasserstoff, oder worin X und Y zusammen für eine Kohlenstoff-Kohlenstoff-Einfachbindung stehen und worin

Z = gegebenenfalls substituiertes Alkyl, gegebenenfalls substituiertes Aryl oder gegebenenfalls substituiertes Hetaryl,

dadurch gekennzeichnet, daß man zu einer Dicarbonsäure der Formel

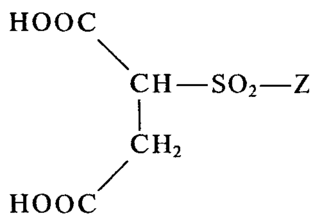

(II)

in welcher

Z die oben angegebene Bedeutung besitzt,

in einem gegenüber Halogenisierungsmittel inerten Lösungsmittel bei 0 bis 80°C mindestens die äquimolare Menge eines Halogenisierungsmittels gibt, und

a) zum Erhalt von Sulfonylcarbonsäuren der Formel (I), in welcher X für Halogen und Y für Wasserstoff steht, das Reaktionsprodukt aufarbeitet oder
b) zum Erhalt von Verbindungen der Formel (I), in welcher X und Y zusammen für eine Kohlenstoff-Kohlenstoff-Einfachbindung stehen, das Reaktionsprodukt auf Temperaturen von 85 bis 150°C erhitzt.

2. Verfahren nach Anspruch 1, dadurch gekennzeichnet, daß in den Verbindungen der Formel (II)

Z für gegebenenfalls durch Alkyl ($C_1-C_4$), Halogen, COOH, $SO_3H$ substituiertes Phenyl; gegebenenfalls durch Halogen, OH, CN, COOH, Alkoxy ($C_1-C_4$), Alkylsulfonyl ($C_1-C_4$), Phenyl, Naphthyl substituiertes Alkyl ($C_1-C_{15}$) steht

und daß es sich bei dem Halogenierungsmittel um Chlor oder Brom handelt.

**Claims**

1. Process for the preparation of sulphonylcarboxylic acids of the general formula

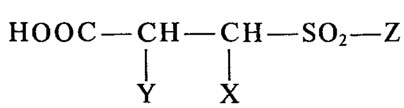

(I)

wherein

X denotes halogen and

Y denotes hydrogen, or wherein X and Y together represent a carbon-carbon single bond, and wherein

Z denotes optionally substituted alkyl, optionally substituted aryl or optionally substituted hetaryl,

characterised in that at least the equimolar amount of a halogenating agent is added to a dicarboxylic acid of the formula

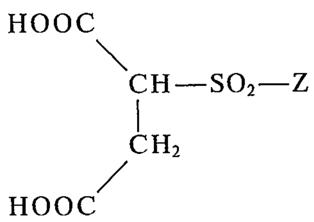

(II)

in which

Z has the abovementioned meaning,

in a solvent which is inert towards the halogenating agent, at 0 to 80°C, and a) in order to obtain sulphonylcarboxylic acids of the formula (I) in which X represents halogen and Y represents hydrogen, the reaction product is worked up, or

b) in order to obtain compounds of the formula (I) in which X and Y together represent a carbon-carbon single bond, the reaction product is heated to temperatures from 85 to 150° C.

2. Process according to Claim 1, characterised in that, in the compounds of the formula (II)

Z represents phenyl which is optionally substituted by alkyl ($C_1$—$C_4$), halogen, COOH or $SO_3H$; or alkyl ($C_1$—$C_{15}$), which is optionally substituted by halogen, OH, CN, COOH, alkoxy ($C_1$—$C_4$), alkylsulphonyl ($C_1$—$C_4$), phenyl or naphthyl

and in that the halogenating agent is chlorine or bromine.

**Revendications**

1. Procédé pour la fabrication d'acides sulfonylcarboxyliques de formule générale

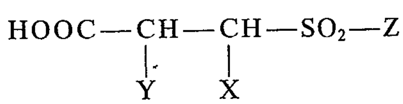

(I)

dans laquelle

X est un halogène et
Y est l'hydrogène ou bien X et Y représentent ensemble une liaison simple carbone-carbone et
Z représente un groupe alkyle éventuellement substitué, aryle éventuellement substitué ou hétaryle éventuellement substitué,

caractérisé en ce que l'on ajoute à un acide dicarboxylique de formule

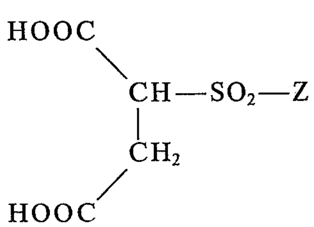

(II)

dans laquelle Z a la signification indiquée ci-dessus, dans un solvant inerte vis-à-vis des agents d'halogénation, à 0—80° C, au moins la quantité équimolaire d'un agent d'halogénation, et

a) pour l'obtention des acides sulfonylcarboxyliques de formule (I), dans laquelle X est un halogène

et Y est l'hydrogène, on traite le produit de réaction ou bien

b) pour l'obtention de composés de formule (I), dans laquelle X et Y, pris ensemble, représentent une liaison simple carbone-carbone, on chauffe le produit de réaction à des températures de réaction de 85 à 150° C.

2. Procédé selon la revendication 1, caractérisé en ce que, dans la formule (II), Z représente un groupe phényle éventuellement substitué par alkyle en $C_1-C_4$, un halogène, COOH, $SO_3H$; un groupe alkyle en $C_1-C_{15}$ éventuellement substitué par un halogène, OH, CN, COOH, alcoxy en $C_1-C_4$, alkylsulfonyle en $C_1-C_4$, phényle, naphtyle; et en ce que l'agent d'halogénation est le chlore ou le brome.